# EUROPEAN PATENT APPLICATION

(11) **EP 0 636 694 A2**
(43) Date of publication of application: **01.02.1995**
(21) Application number: 94111952.1
(22) Date of filing: 01.08.1994
(51) Int. Cl.: C12N 15/16, C07K 14/66

(54) **Human and human-like thymopoietin, method for its production and recombinant DNA sequences encoding it**

(30) Priority: 01.08.1993 IL 10654593
(71) Applicant: Q.B.I. ENTERPRISES LIMITED, Omer 84965 (IL)
(72) Inventor: Zurr, Daniel, Herzliya (IS)
(74) Representative: VOSSIUS & PARTNER

(57) **Abstract**

A DNA construct comprising a DNA sequence which encodes for human or human-like thymopoietin, the said DNA sequence coding for an amino acid sequence substantially the same as:

### SEQUENCE LISTING

(1) GENERAL INFORMATION:
   (i) APPLICANT:
      (A) NAME: Q.B.I. ENTERPRISES LTD.
      (B) STREET: 38, TAMAR ST.
      (C) CITY: OMER
      (E) COUNTRY: ISRAEL
      (F) POSTAL CODE (ZIP): 84965
   (ii) TITLE OF INVENTION: HUMAN AND HUMAN-LIKE THYMOPOIETIN, METHOD FOR ITS PRODUCTION AND RECOMBINANT DNA SEQUENCES ENCODING IT.
   (iii) NUMBER OF SEQUENCES: 7
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA:
      APPLICATION NUMBER: EP 94 11 1952.1
   (vi) PRIOR APPLICATION DATA:
      (A) APPLICATION NUMBER: IL 106545
      (B) FILING DATE: 01-AUG-1993
(2) INFORMATION FOR SEQ ID NO: 1:
   (i) SEQUENCE CHARACTERISTICS:
      (A) LENGTH: 2514 base pairs
      (B) TYPE: nucleic acid
      (C) STRANDEDNESS: double
      (D) TOPOLOGY: unknown
   (ii) MOLECULE TYPE: DNA (genomic)
   (vi) ORIGINAL SOURCE:
      (A) ORGANISM: MURINE THYMOPOIETIN GENE
   (ix) FEATURE:
      (A) NAME/KEY: CDS
      (B) LOCATION: 213..362
   (xi) SEQUENCE DESCRIPTION: SEQ ID NO: 1:
   CGAGCCCGCA GCCGTGCGCC AGGGGCTTTT TGTGGCGGGG GCCGGACCTG GGTTTTGTGT        60
   CCCGAGTTCT GTGCCGTGCC CGCGGCGCCG CTCCGGGGCA GGGATCTCCC GAGGCGGCGG       120
   GCGCAGCCCG GGACCAGCGA GCGAGCGCGC CGGCGTGAGC AGCGGCGGCG GCGACTGTGA       180
   GGGGCCGGGG AGGAGAGAAG GAGGGGGACG AG ATG CCG GAG TTC CTA GAG GAC         233
                                       Met Pro Glu Phe Leu Glu Asp
                                         1               5
   CCT TCG GTC CTG ACC AAA GAC AAG TTG AAG AGC GAG TTG GTC GCC AAC      281
   Pro Ser Val Leu Thr Lys Asp Lys Leu Lys Ser Glu Leu Val Ala Asn
           10                  15                  20
   AAC GTG ACG CTC CCG GCC GGC GAG CAG CGC AAG GAC GTG TAC GTG CAG      329
   Asn Val Thr Leu Pro Ala Gly Glu Gln Arg Lys Asp Val Tyr Val Gln
       25                  30                  35
   CTC TAC CTG CAG CAC CTC ACG GCG CGC AAC CGG CCGCCGCTCG CCGCGGGAGC    382
   Leu Tyr Leu Gln His Leu Thr Ala Arg Asn Arg
    40                  45                  50
   CAACAGCAAA GGGCCGCCCG ACTTCTCGAG CGACGAGGAG CGCGACGCCA CGCCGGTGCT    442
   CGGCTCCGGG CCTCCGTGGG TCGCGGCCGC GCGCCGTCGG CAGGAAAGCC ACAAAGAAAA    502
   CTGATAAGCC CAGGCTAGAA GATAAAGATG ATCTGGATGT GACAGAGCTC TCTAATGAAG    562
   AACTTCTGGA TCAGCTTGTA AGATATGGAG TGAATCCTGG TCCCATTGTG GGAACAACCA    622
   GGAAGCTATA TGAGAAGAAG CTGTTGAAGC TGAGGGAGCA GGGAACTGAA TCGAGATCCT    682
   CTACTCCTCT TCCAACAGTC TCTTCCTCTG CAGAAAACAC AAGGCAGAAT GGAAGTAACG    742
   ACTCTGACAG ATACAGCGAC AATGATGAAG ACTCTAAAAT AGAGCTGAAG CTTGAGAAGA    802
   GGGAGCCGCT AAAGGGCAGA CGAAGACGCC AGCTACACTG AAGCAGAGAA GGACTGAGCA    862
   CAATCAGGTA TTTGTAGTTT TATGATCCCC GTGTCCAGGT GTGTCATCTG ACTAGTATCA    922
   TCCACCCCAT GTCTTGACCT TTGGTGCACA CACGCCGTGC AGCCTGCTCA CCCCACTGAC    982
   TGGATTCATG GTTGAGTGGT GTGGGGCACA TACTTTGTGA GATGTGTCAT CAGCATAGCC   1042
   TCCGTTGTTT ATTGGATTTA AGTAGTCTAG CATTAGTCAT GCTGCCTGTT TCTGATTTGG   1102
   GCTCAGCAGT TTTGAACCTG GGGTCTCGGG TATGGCAGGC CAGCCTTCCA CCCTCCTGCT   1162
   TCTTCCTCTT GAGATGATTG CTTTAGAAGA TAAAATGCAC CCTTTTTGAT GCAACTTTGA   1222
   AAGCTCGGGA AAGTTTCATT TGTGTCTAGA TTTCTCAGTT GAATTTCCTT TATTTGTAGC   1282
   TTCTTAACAC TCTGAAGCTA GCTAGTATGG TACATTATTT AAAATGACAA TTTCCTCTTT   1342
   TGGGGGGATG GGACAGGAAG GGCAAGGTAG AGTTTTCAAA AGAAAACTGG GGAGAGAATG   1402
   AGCATAGGCA TCAATAAAGA CCTTCTTTTA CAGAGGGAAA TGACTGGCTC TGATTTAGCC   1462
   TCCACACCCT AGGTTCTTTT CCTCATCTTT ACTCGTTAGC TTGAAAATGT GTCAGTTTGT   1522
   TGTTCATCTA TCTTTTCCAT GTTCTGTCTG CCTCTGGTTT CTGTGGTGTT TTGAAACAGT   1582
   ACTTCACAAT GAAGCCCTGG CCTGCCTGAC CAGGCTGGTC TCTAACTTGT AGAGCTTTCT   1642
   GCTTCCTGAG AGATGGGATT ACAGAGATGG GATCCCAGTT GTGCACCGAC AGTCCTGCTT   1702
   CCTTCCTTCC GTGTGATCGG AGGGGAGAGG CAGGGTCCTG TTCTCCTGAC CCTTTACAGG   1762
   ACTACAGGAG TGTACCACCA ATCCTGGCCA AAAAATGGTT TACTGCATAA TTAAGTTGAG 1822
   TCTGAACAAT ACGTCTATAC TAATTCCCAC CGTGGCTGTT TCTCCGTCCT CCGTTTGGCG 1882
   ACCGTAGTTA ACCATGTCGG TATTTCTGCT TGCTGCAGGT GGCAGGTGGT TTTCAAGGGT 1942
   TTTATCTGTT TCTTGATACC TGTGTAACTG GCATACTGGG GCTAGACCTG CATGTGGCAG 2002
   TGCAGAGACT TGGGCCTTTT TTCCTTTTGT AGTCACAATA AAAATGTTTG TCAGTTCTGG 2062
   AGTAGTCTCG TTTTAACTGT TAGAGTTAGA CTATGGACTC CTATCTATTT AAATAGAACT 2122
   ATGGAGCTGG TGACTACTGT ATTGGACAGC TTAGCCCTAA AGGGTTTGTC TGGGTTTGGG 2182
   TTCAGTTTCT TTGAAAAGAA TACTTCATAA ATGATGTTGT AGCCTTCCTA CTGATCATTT 2242
   CAGGAAATAT GAATTTCCTT CAAAACTATT AAAATTAATT AATAGTTCAG CTGTTGGTCT 2302
   TTTCAGTTAA GGATTTTAAC AGCTGTGAAT AATACTGCCT AAACCCGTGA CTTTATTATA 2362
   GTCACAAAGT GATGACTTCT CACCATGTGA TTTGATATCT GTGACTGATT ACCTCATGTT 2422
   CCTGGGTAAG ATCCTTAGCT GCCTTAAAGT TAAGTTCAAA TAAAACAGCT AGGAAAAACT 2482
   GGATTTGAGA TGCGGTTTCT CTGTGGGAAT TC 2514
(2) INFORMATION FOR SEQ ID NO: 2:
   (i) SEQUENCE CHARACTERISTICS:
      (A) LENGTH: 50 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Pro Glu Phe Leu Glu Asp Pro Ser Val Leu Thr Lys Asp Lys Leu
   1 5 10 15
   Lys Ser Glu Leu Val Ala Asn Asn Val Thr Leu Pro Ala Gly Glu Gln
   20 25 30
   Arg Lys Asp Val Tyr Val Gln Leu Tyr Leu Gln His Leu Thr Ala Arg
   35 40 45
   Asn Arg
   50
(2) INFORMATION FOR SEQ ID NO: 3:
   (i) SEQUENCE CHARACTERISTICS:
      (A) LENGTH: 150 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: HUMAN THYMOPOIETIN
   (ix) FEATURE:
      (A) NAME/KEY: CDS
      (B) LOCATION: 1..150
   (xi) SEQUENCE DESCRIPTION: SEQ ID NO: 3:
   ATG CCG GAG TTC CTG GAA GAC CCC TCG GTC CTG ACA AAA GAC AAG TTG        48
   Met Pro Glu Phe Leu Glu Asp Pro Ser Val Leu Thr Lys Asp Lys Leu
     1               5                  10                  15
   AAG AGT GAG TTG GTC GCC AAC AAT GTG ACG CTG CCG GCC GGG GAG CAG        96
   Lys Ser Glu Leu Val Ala Asn Asn Val Thr Leu Pro Ala Gly Glu Gln
                20                  25                  30
   CGC AAA GAC GTG TAC GTC CAG CTC TAC CTG CAG CAC CTC ACG GCG CGC       144
   Arg Lys Asp Val Tyr Val Gln Leu Tyr Leu Gln His Leu Thr Ala Arg
            35                  40                  45
   AAC CGG                                                               150
   Asn Arg
        50
(2) INFORMATION FOR SEQ ID NO: 4:
   (i) SEQUENCE CHARACTERISTICS:
      (A) LENGTH: 50 amino acids
      (B) TYPE: amino acid
      (D) TOPOLOGY: linear
   (ii) MOLECULE TYPE: protein
   (xi) SEQUENCE DESCRIPTION: SEQ ID NO: 4:
   Met Pro Glu Phe Leu Glu Asp Pro Ser Val Leu Thr Lys Asp Lys Leu
     1               5                  10                  15
   Lys Ser Glu Leu Val Ala Asn Asn Val Thr Leu Pro Ala Gly Glu Gln
                20                  25                  30
   Arg Lys Asp Val Tyr Val Gln Leu Tyr Leu Gln His Leu Thr Ala Arg
            35                  40                  45
   Asn Arg
        50
(2) INFORMATION FOR SEQ ID NO: 5:
   (i) SEQUENCE CHARACTERISTICS:
      (A) LENGTH: 150 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: MURINE THYMOPOIETIN
   (xi) SEQUENCE DESCRIPTION: SEQ ID NO: 5:
   ATGCCGGAGT TCCTAGAGGA CCCTTCGGTC CTGACCAAAG ACAAGTTGAA GAGCGAGTTG      60
   GTCGCCAACA ACGTGACGCT CCCGGCCGGC GAGCAGCGCA AGGACGTGTA CGTGCAGCTC     120
   TACCTGCAGC ACCTCACGGC GCGCAACCGG                                      150
(2) INFORMATION FOR SEQ ID NO: 6:
   (i) SEQUENCE CHARACTERISTICS:
      (A) LENGTH: 150 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: BOVINE THYMOPOIETIN
   (ix) FEATURE:
      (A) NAME/KEY: CDS
      (B) LOCATION: 1..150
   (xi) SEQUENCE DESCRIPTION: SEQ ID NO: 6:
   ATG CCG GAG TTC CTG GAA GAC CCC TCG GTC CTG ACG AAA GAG AAG TTG       48
   Met Pro Glu Phe Leu Glu Asp Pro Ser Val Leu Thr Lys Glu Lys Leu
     1               5                  10                  15
   AAG AGT GAG TTG GTC GCC AAC AAC GTG ACG CTC CCG GCC GGG GAG CAG       96
   Lys Ser Glu Leu Val Ala Asn Asn Val Thr Leu Pro Ala Gly Glu Gln
                20                  25                  30
   CGC AAA GAC GTG TAT GTG CAG CTC TAC CTG CAG CAC CTC ACG GCG CGC      144
   Arg Lys Asp Val Tyr Val Gln Leu Tyr Leu Gln His Leu Thr Ala Arg
            35                  40                  45
   AAC CGG                                                              150
   Asn Arg
        50
(2) INFORMATION FOR SEQ ID NO: 7:
   (i) SEQUENCE CHARACTERISTICS:
      (A) LENGTH: 50 amino acids
      (B) TYPE: amino acid
      (D) TOPOLOGY: linear
   (ii) MOLECULE TYPE: protein
   (xi) SEQUENCE DESCRIPTION: SEQ ID NO: 7:
   Met Pro Glu Phe Leu Glu Asp Pro Ser Val Leu Thr Lys Glu Lys Leu
     1               5                  10                  15
   Lys Ser Glu Leu Val Ala Asn Asn Val Thr Leu Pro Ala Gly Glu Gln
               20                  25                  30
   Arg Lys Asp Val Tyr Val Gln Leu Tyr Leu Gln His Leu Thr Ala Arg
           35                  40                  45
   Asn Arg
       50

## Description

The present invention relates to the production of thymopoietin. More particularly, the invention relates to methods for producing human or human-like thymopoietin, and to means for such production.

Thymopoietin (TP) is a hormone-like material which is believed to be of importance in a variety of biological processes. TP is a humoral factor secreted by the thymus. Other thymic factors have been isolated, mostly from calf thymuses, through extraction and purification procedures. Several peptides which are unrelated in amino acid content and sequence have been completely purified and sequenced, and include, besides thymopoietin, also thymulin, THF and several thymosin peptides.

TP plays a major role in enhancing T-cells maturation and activation. TP is known to have an important influence on the immune system, and in particular it appears to have beneficial clinical effects in conditions of immunodeficiency, cancer, infectious diseases and autoimmuno disorders, such as rheumatoid arthritis. For this reason, many efforts have been made in the art to provide thymopoietin, which so far have had only limited success, because the preparation of full length TP by extraction and purification, or by synthesis, is impractical and cannot be used for routine preparation of this compound for medical uses, and because the hTP sequence was not known.

It is also known that a short peptide synthesized on the basis of a known sequence, the 32-36 amino acid sequence of bovine TP contains the active site of thymopoietin. This peptide, called thymopentin, is however instable, exhibiting an extremely short half-life in vitro, and cannot be used as such for this reason. It appears that the whole thymopoietin peptide is needed in order to impart stability to the thymopentin active fragment.

Bovine thymopoietin has been cloned as described in European Patent Application No. 92300671.2. This application discloses the nucleotide sequence encoding the 18 to 49 amino acids of bovine thymopoietin. However, bovine thymopoietin differs from human thymopoietin, as more fully discussed below. This difference renders undesirable the use of bovine thymopoietin as a therapeutical agent in humans, because of the expected adverse immune response of the human body to it.

Audhya et al. published in 1987 an article entitled "Isolation and complete amino acid sequence of human thymopoietin and splenin" [Proc. Natl. Acad. Sci. USA Vol. 84, pp. 3545-3549, June 1987], in which the amino acid sequences of the bovine and human thymopoietin were given. Unfortunately, both sequences presented in this article were wrong and differ in several amino acids from the actual sequences. This publication taught away from the correct sequences and thus rendered the way to the present invention more difficult.

The present invention now provides the sequence and means to produce human or human-like thymopoietin. It has been surprisingly found, and this is an object of the invention, that while the nucleotides coding for thymopoietin in the murine and in the human genes are different in 10 positions, the wobbeling effect of the murine coding sequence is such that the resulting peptide is the same as that produced by the human gene.

As will be appreciated by the skilled person, different nucleotide sequences can code for identical peptides, as is the case with human and murine coding sequences. The skilled person will recognise additional coding sequences which will provide the exact amino acids sequence of human TP. In this context, throughout the specification reference is made to "human thymopoietin", when the peptide is produced by a DNA sequence essentially the same as the coding sequence of the human gene, and to "human-like thymopoietin" when the peptide is produced by a DNA sequence which is different from the human DNA sequence, but which codes for essentially the same amino acid sequence as that of human TP, which sequence may be, for instance, the coding sequence of the murine gene.

In one aspect, the invention is directed to a DNA construct comprising a DNA sequence which encodes for human or human-like thymopoietin, the said DNA sequence coding for an amino acid sequence substantially the same as:

In another aspect, the invention is directed to a DNA construct comprising a DNA strand sequence encoding for TP, the same as:

In still another aspect, the invention is directed to a DNA construct comprising a DNA strand sequence encoding for TP, the same as:

Also encompassed by the invention is a host cell capable of expression of human or human-like thymopoietin comprising one of the aforesaid DNA constructs, and transcriptional and translational DNA sequences positioned in relation to the thymopoietin sequence to direct expression of thymopoietin.

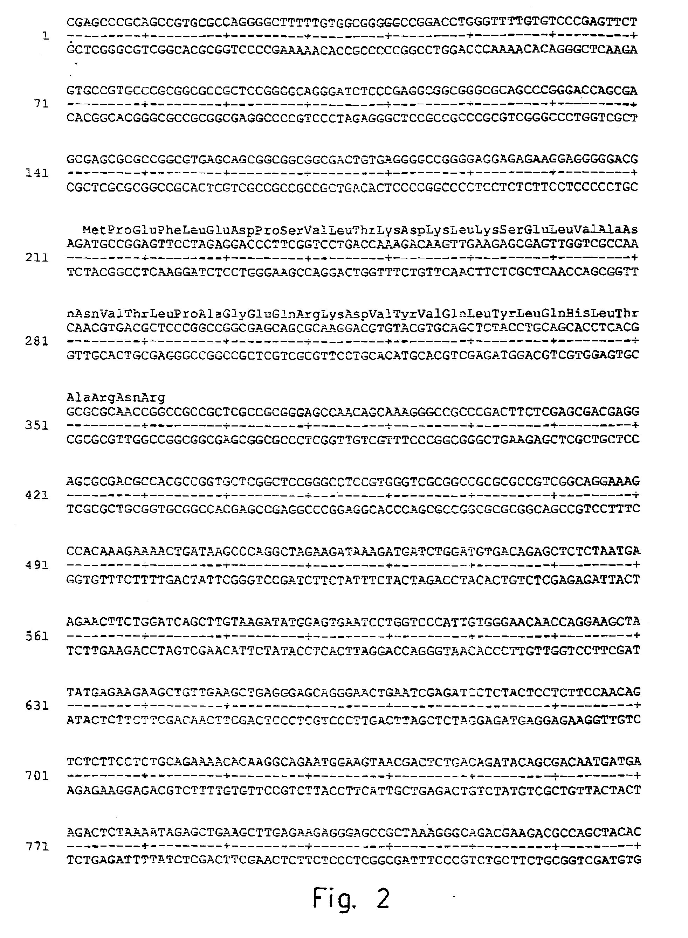

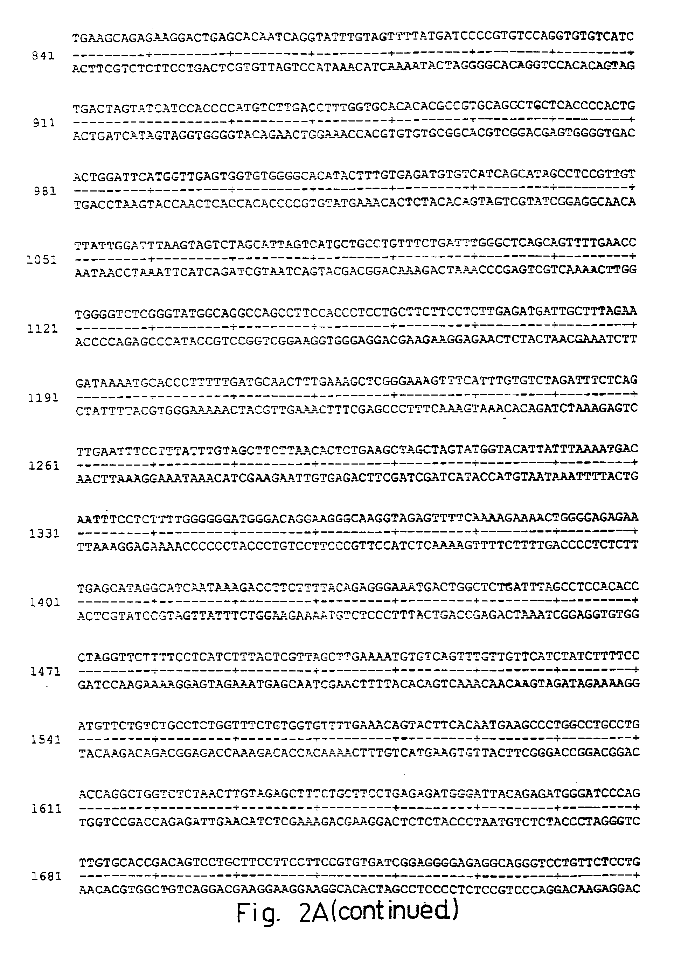

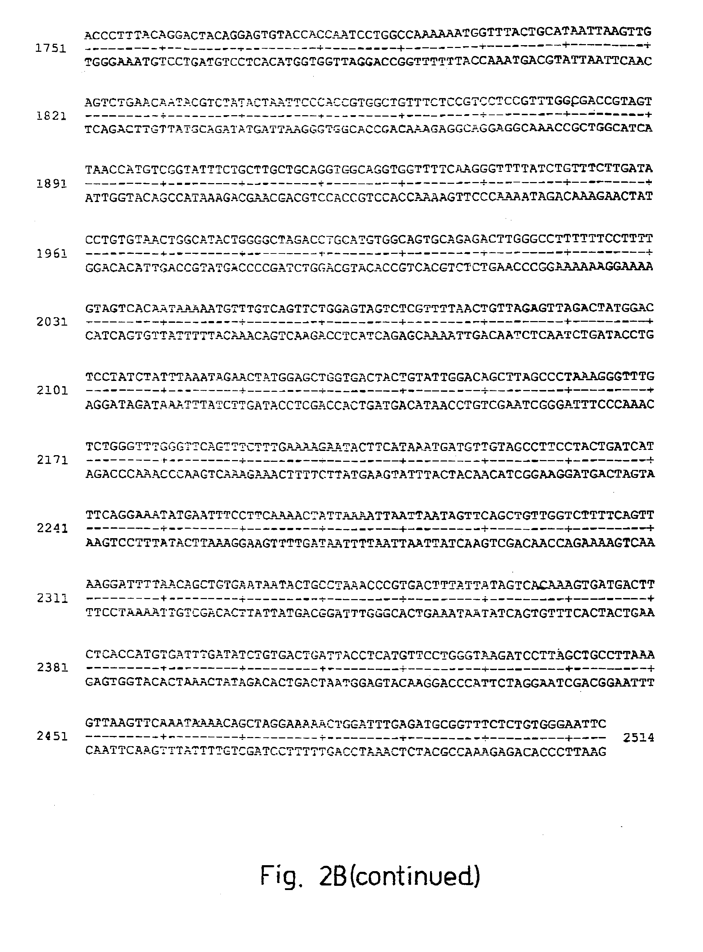

As will be appreciated by the skilled person, while streamlined DNA constructs can be conveniently provided, by utilizing the coding sequence encoding the first 49 amino acids which constitute thymopoietin, it is also possible to use the whole thymopoietin gene, since cleavage of the resulting polypeptide, to give thymopoietin, is obtained by the presence of appropriate enzymes. Accordingly, in one embodiment of the invention the DNA construct comprises the murine thymopoietin gene of Fig. 2.

The invention is further directed to a method of producing human or human-like thymopoietin, which method comprises providing a DNA construct comprising a DNA sequence coding for an amino acid sequence substantially the same as:
and expressing the same in a suitable host cell.

According to a preferred embodiment of the invention the DNA strand sequence encoding TP is the same as:

According to another preferred embodiment of the invention the DNA strand sequence encoding TP is the same as:

In one embodiment of the invention the human-like thymopoietin is murine thymopoietin.
Fig. 1 is a schematic representation of the murine thymopoietin cDNA clone;
Fig. 2 is the detailed sequence of the murine thymopoietin gene;
Fig. 3 shows the human thymopoietin coding sequence and relative amino acids;
Fig. 4 shows a comparison of the human, bovine and murine thymopoietin coding sequences; and
Fig. 5 shows a comparison of the human, bovine and murine thymopoietin peptides.

The examples illustrate the invention.

### Experimental Methods

Standard laboratory procedures are not discussed in detail, for the sake of brevity, and they were carried out according to Sambrook, J. et al., Molecular Cloning, A Laboratory Manual, New York: Cold Spring Harbor Laboratory Press, 1989, and Ausubel, FM et al. (eds), (1993), Current Protocols in Molecular Biology, Green Publishing and Wiley-Interscience, New York, unless otherwise stated.

Restriction and modification enzymes were bought from New England Biolabs, Boehringer Mannheim, Bethesda Research Laboratories and Stratagene Cloning Systems. The conditions for all enzymatic reactions were according to the manufacturer instructions.

### Isolation of Murine Thymopoietin (TP) cDNA (Gene)

A mouse cDNA library (Stratagene Cloning Systems) was screened with a bovine TP probe (a 126 bp EcoRl-Pstl segment of the bovine TP coding sequence). 10⁶ clones were screened and five reacting clones were isolated.

The largest cDNA clone, 2.5 Kb long, was characterized with restriction enzymes. Several overlapping fragments of it were subcloned into bluescript plasmid (Stratagene Cloning Systems) and were isolated. The full nucleotide sequence was determined on the two DNA strands using a Sequenase version II sequencing kit (USB - United States Biochemicals, Cleveland, Ohio, U.S.A.) and T7, T3, as well as specific TP primers. T7 (Cat. # 1227) and T3 (Cat. # 1228) primers were purchased from New England Biolabs, U.S.A.

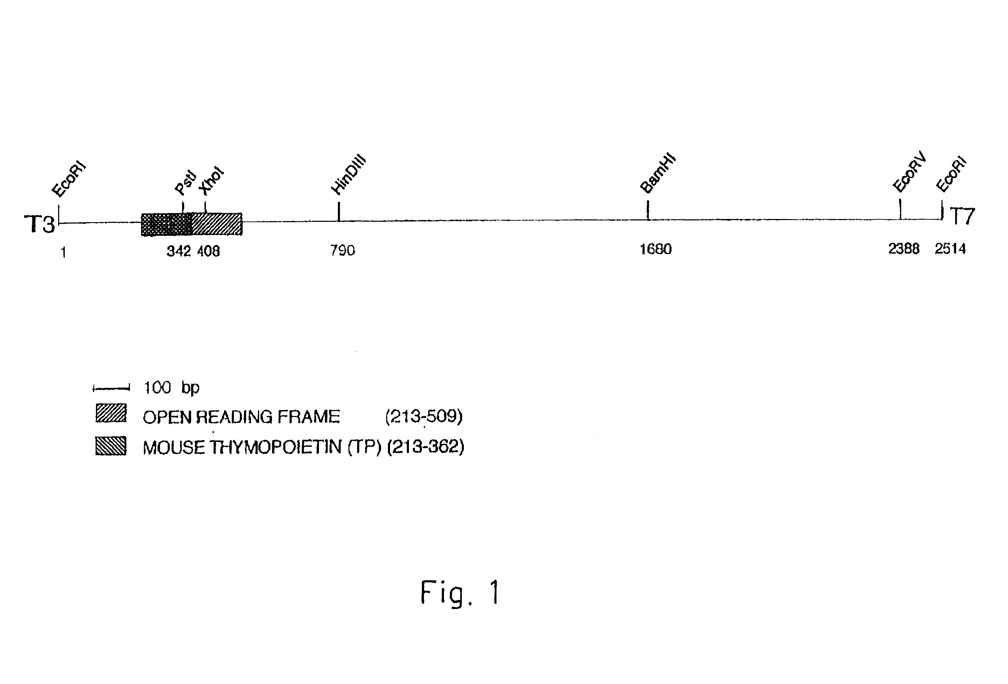

The clone is schematically shown in Fig. 1.

### Determination of the Murine TP Coding Sequence

The putative amino acid sequence of the murine cDNA clone was determined by computer analysis. Starting at the first AUG codon at nucleotide 213, there is an open reading frame coding for 97 amino acids. Nucleotides 213-362 are coding for the mouse TP polypeptides. Comparison of the murine clone with the bovine TP nucleotides and amino acid sequences showed that translation from the first AUG gives a peptide of which the first 49 amino acids are very similar to the published bovine TP amino-acid sequence. The next 47 amino acids also show similarity to bovine TP, however to a less extent. The only difference in the TP sequence (first 49 amino acids) is in amino acid 14: Asp in the case of mouse, and Glu in the case of bovine. The following 47 amino acids which reside 3' to the TP did not show any significant homology to any known peptide. The mouse nucleotide sequence 3' to the coding region is not homologous to the 3' of the bovine cDNA. Moreover, the 3' of the TP coding sequence of bovine, murine and human are the same: Arg-Pro. Comparison of the nucleic-acid sequences of mouse and bovine shows nine differences between the two sequences.

The full sequence of the murine TP gene is shown in Fig. 2.

### Determination of the Human TP Coding Sequence

Oligonucleotide primers based on the sequence of the mouse cDNA clone were comprised. The following primers were prepared:
1) Nucleotides 201-217
2) Nucleotides 402-420

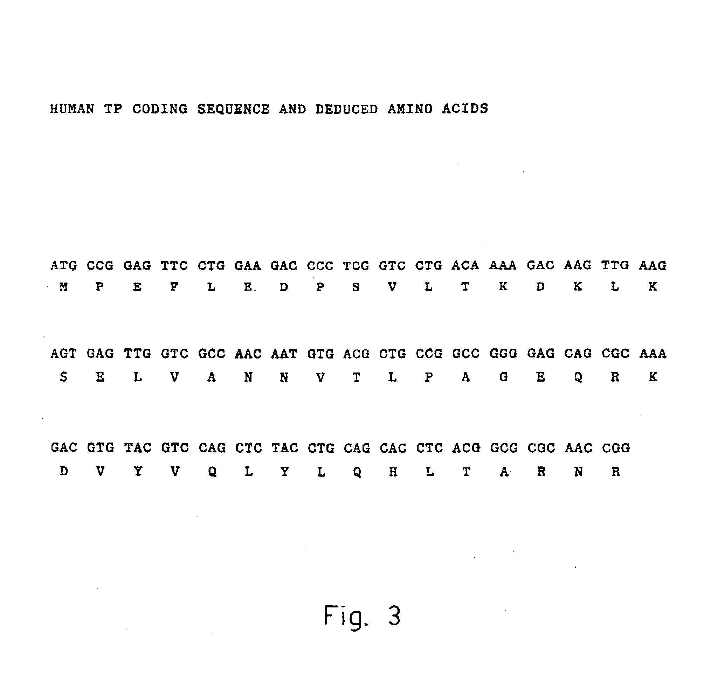

Human DNA was prepared from blood lymphocytes. The TP coding sequence was isolated by PCR amplification using the above-mentioned primers. The amplified segment was cloned into pCRII plasmid (Invitrogen, San Diego, Cal., U.S.A.). The nucleotide sequence of this segment was determined as described above with respect to the murine sequence, using a Sequenase version II sequencing kit from USB and primers specific for the plasmid (T7 and T3). This sequence is shown in Fig. 3.

### Example 1

### Expression of Mouse TP cDNA (Gene) Clone in CHO Cells

The full cDNA clone of the TP was subcloned into the expression vector pRc/CMv (Invitrogen). For this 1µg of the pRC/CMV DNA was cut with 10 units of each BstXl and Apal restriction enzymes (Boehringer Manheim) which reside in the cloning vector but not in the TP gene. 3αg of the TP plasmid (in pBluescript) were cut as well. The insert was separated on 1 % agarose gel. Following gel electrophoresis the relevant band was sliced out of the gel and was cleaned using Jetsorb (Genomed GmbH, Germany). The insert was ligated to the vector using T₄ ligase (New England Biolabs) and 10:1 molar ration of insert to vector. The construct was electrotransfected into Chinese hamster ovary (CHO) cells, available from the American Type Culture Collection (ATCC). For electrotransfection EASYJECT (from Equibio, Belgium) was used in twin pulse bode. 10⁶ cells were suspended in 800 µl DMEM supplemented with 10% fetal calf serum, and were incubated for 1 minute at room temperature with 10 µg of purified DNA prior to the electrotransfection. The pulse electrical parameters were: 650 volts, 25 µF and 99 Ohms for the first pulse, inter pulse time 0.1 seconds, and 100 volts, 1500 µF and 99 Ohms for the second pulse.

A G418 selection (Southern, P.J. and Berg, P., (1982), J. Mol. Appl. Gen. _1, 327-341) was applied. CHO cells expressing the Neo resistant gene were selected by growth in DMEM medium containing 10% fetal calf serum and 800 µg/ml G-418. Individual clones were picked up and were analysed for the level of TP production. High productive clones were selected.

Cells supernatants as well as cell lysates of stable clones were collected and assayed for TP expression, using the bioassay method specific for TP described by Audhya, T. and Goldstein, G., Methods in Enzymology 116, 279-291 (1985); and Scheid, M.P. et al., J. Exp. Med. 147, 1727-1743 (1978).

Best expressing clones were chosen. TP expression in the CHO was enhanced in the usual methods (Ausubel et al., Unit 16.14). The best stable clones gave a yield of 10-50 µg/ml of TP. The TP obtained was analyzed and was found to be identical to human TP.

### Example 2

### Expression of Mouse TP in CHO Cells

Using oligonucleotides as primers the 147bp fragment coding for the TP 49 amino acids was amplified by PCR and was cloned into the TA cloning vector (Invitrogen) according to the manufacturer's instructions. The TP insert was subcloned into the pRC/CMV expression vector using the same restriction enzymes as in Example 1, but this time the insert was isolated from 2% agarose gel.

The expressing vector was electrotransfected into CHO cells and stable cell clones expressing TP were selected in the same procedure as in Example 1.

The TP obtained was analyzed and was found to be identical to that obtained using the full murine TP gene (as well as to human TP).

### Example 3

### Expression of Human TP Coding Sequence in CHO Cells

The human TP clone obtained as described above was subcloned into pRC/CMV expression vector. CHO cells were electrotransfected with the construct, as described in Example 1. Stable Neomycin resistance cells were isolated, and expression of TP by different clones was examined by the bioassay specific for TP, as described in Example 1.

### Example 4

### Expression of Murine TP In Thymus (In Situ Hybridization)

In situ hybridization (Ausuber et al., ibid (1993), Unit 14) was done on mouse thymic cross-section. Murine TP synthetic anti-sense and sense (as a negative control) were prepared. The probes were labeled with DIG (Boehringer Manheim kit, Cat. # 1175025). Upon detection, it was found that the TP RNA is expressed mainly in the outer thymic cortex and to a lesser extent in the medula. This experiment shows that TP according to the invention is normally expressed in the thymus.

As will be apparent to the skilled person, the invention permits to produce human or human-like thymopoietin, utilizing the appropriate coding sequence derived from the human or the murine gene. The invention further permits to produce human-like TP using coding sequences different from the murine sequence, but which produce an essentially identical TP.

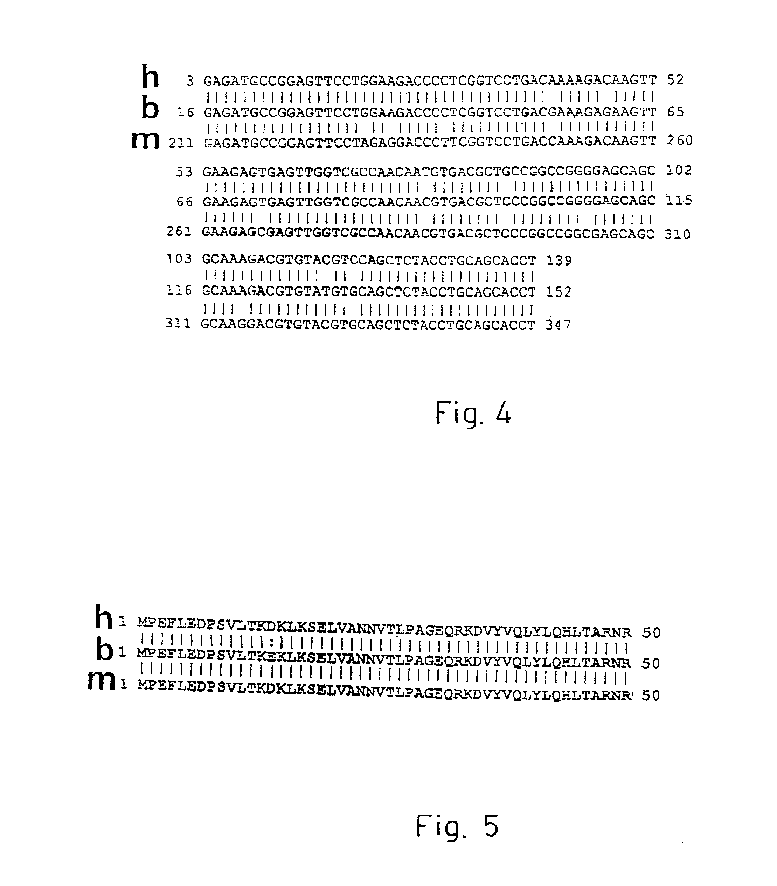

The human (h), bovine (b) and murine (m) TP coding sequences are compared in Fig. 4, from which the differences (missing bar, relative to the uppermost sequence) can be easily seen. The TPs obtained from the coding sequences of Fig. 4 are compared in Fig. 5. It can be seen that the 14th amino acid of the bovine peptide (E) differs from the human and murine amino acid (D), as shown by the dotted line.

It should further be noted that the invention is not limited to peptides comprising the TP sequence only. Addition of amino acids is also encompassed by the invention, as long as the basic TP function and structure is maintained.

## Claims

1. A DNA construct comprising a DNA sequence which codes for human or human-like thymopoietin, the said DNA sequence coding for an amino acid sequence substantially the same as:

2. The DNA construct according to claim 1, wherein the DNA strand sequence coding for TP is:

3. The DNA construct according to claim 1, wherein the DNA strand sequence coding for TP is:

4. The construct of claim 3 wherein the human-like thymopoietin is murine thymopoietin.

5. A host cell capable of expression of human or human-like thymopoietin comprising the DNA construct of any of claims 1 to 4, and transcriptional and translational DNA sequences positioned in relation to the thymopoietin sequence to direct expression of thymopoietin.

6. A DNA construct comprising a DNA sequence which codes for a human or human-like thymopoietin, the said DNA sequence being derived from mice.

7. The DNA construct according to claim 6, comprising the murine thymopoietin gene of Fig. 2.

8. A method of producing human or human-like thymopoietin, comprising providing a DNA construct comprising a DNA sequence coding for an amino acid sequence substantially the same as:
and expressing the same in a suitable host cell.

9. The method according to claim 8, wherein the DNA strand sequence coding for TP is:

10. The method according to claim 8, wherein the DNA strand sequence coding for TP is:

11. The method according to claim 10 wherein the human-like thymopoietin is murine thymopoietin.

12. A method of producing human-like thymopoietin, comprising providing a DNA construct comprising the murine thymopoietin gene of Fig. 2, and expressing the same in a suitable host cell.

13. A synthetic thymopoietin, comprising an amino acid sequence substantially the same as:
